# EUROPEAN PATENT SPECIFICATION

(11) **EP 2 117 534 B1**
(45) Date of publication and mention of the grant of the patent: **05.10.2011**
(21) Application number: 08734289.5
(22) Date of filing: 03.03.2008
(51) Int. Cl.: A61K 31/40, A61K 9/16, A61K 9/28

(54) **A PHARMACEUTICAL COMPOSITION WITH ATORVASTATIN ACTIVE INGREDIENT**
PHARMAZEUTISCHE ZUSAMMENSETZUNG MIT ATORVASTATIN ALS WIRKSTOFF
COMPOSITION PHARMACEUTIQUE À PRINCIPE ACTIF ATORVASTATINE

(30) Priority: 02.03.2007 CZ 20070169
(43) Date of publication of application: 18.11.2009
(73) Proprietor: Zentiva, K.S., 102 37 Praha 10 (CZ)
(72) Inventor: PROKOPOVA, Alena, 101 00 Praha 10 (CZ); SEBEK, Pavel, 161 00 Praha 6 (CZ); DUBOVSKA, Michaela, 101 00 Praha 10 (CZ); TOMASEK, Vaclav, 130 00 Praha 3 (CZ); HANOVSKA Anna, 14900 Praha 11 (CZ)
(74) Representative: Jirotkova, Ivana
(86) International application number: PCT/CZ2008/000024
(87) International publication number: WO 2008/106901

(56) References cited:
- EP-A- 1 336 405
- EP-A- 1 808 162
- EP-A- 1 810 667
- WO-A-2007/118873
- US-A1- 2004 247 673
- US-A1- 2007 014 846
- PFIZER: "Lipitor"[Online] June 2006 (2006-06), XP002488210 Retrieved from the Internet: URL:http://www.pfizer.com/files/products/p pi_lipitor.pdf> [retrieved on 2008-07-15]

## Description

### Technical Field

The invention relates to a pharmaceutical composition containing a substance with well-known anti-hyperlimidemic effects - atorvastatin. The composition has excellent properties both from the point of view of physical characteristics and as regards the speed of releasing the active substance or product stability.

### Background Art

The hemi-calcium salt of (3*R*,5*R*) 7-[3-phenyl-4-phenylcarbamoyl-2-(4-fluorophenyl)-5-isopropylpyrrol-1-yl]-3,5-dihydroxyheptanoic acid of formula I 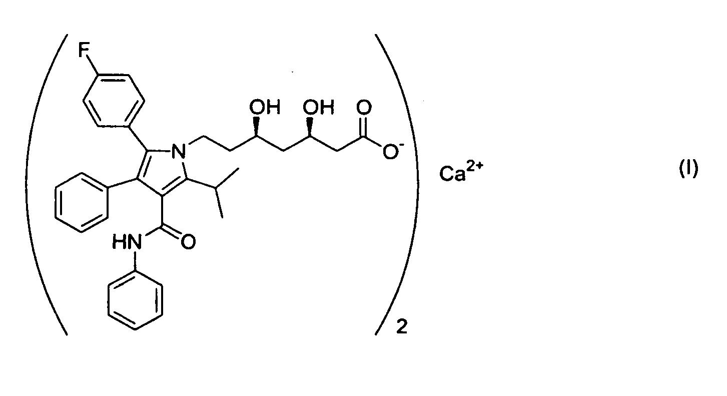 known under the non-proprietary name atorvastatin (I), possibly also the calcium salt of atorvastatin in the text below, is produced in accordance with published patents (US patents 4,681,893 and 5,273,995). This medicament is an important representative of hypo-lipidemic and hypo-cholesteric medicaments.

Atorvastatin belongs to the group of anti-hyperlipidemically effective substances described in the patent specification No. EP 247633.

In particular, the calcium salt of atorvastatin and its specific method of preparation were described in the patent specification No. EP 409281.

A number of other patent applications have dealt with the preparation method of atorvastatin in its various crystalline or amorphous forms.

Patent applications WO 9703958 and WO 9703959, describing stable crystalline forms of the calcium salt, and WO 9703960 A, describing the process of their transformation to an amorphous substance, are worth mentioning.

The last mentioned application shows that the amorphous product is desirable from the point of view of the speed of releasing of the active substance from the pharmaceutical composition. It may be different in the case of crystals, which would bring uncertainty into the final effect of the atorvastatin-containing product.

Atorvastatin, including its salts, mainly its calcium salt, which is used in commercial products, has turned out to be a substance with problematic stability. In the case of using the amorphous form of atorvastatin the problem of stability is even more urgent (WO 9703958).

Patent application No. WO 9416693 describes several influences that may have a negative impact on stability of a pharmaceutical composition of atorvastatin, namely heat, acidic environment, humidity and light.

In accordance with the above mentioned application, stabilization of the pharmaceutical composition is achieved by addition of basic salts of Ca, Mg or Li ions. This way the above mentioned negative influences are eliminated. However, the stability values of the pharmaceutical composition mentioned in this application approximate the limit of acceptability and do not comply with increasing requirements for the quality of pharmaceutical products.

However, application No. WO 0035425 shows that the use of a too basic reagent may cause local irritation of mucous membranes in the digestive tract, which may lead to indigestion in patients. This is why the application recommends replacing the previously known salts with the finer buffers. The stability of the products is allegedly good and a minimum quantity of degradation products has been found. The application does not contain particular data of their quantity and methods of their measurement.

However, application No. WO 02072073 shows that too low pH impairs releasing of the active substance. Especially, if one considers the acidic environment of the stomach, the buffers suggested in the previous application would have to be present in a very high concentration. The composition in accordance with the quoted application should, after dissolution in simulated gastric juice, increase pH to pKa + 1 of atorvastatin acid. This can be achieved either with a large quantity of the buffer requiring a very large tablet or by replacing the buffer with a stronger base, e.g. MgO, which however involves the possibility of local mucous membrane irritation.

The described invention deals with such a composition that reduces the possibility of mucous membrane irritation, achieves excellent values of releasing the effective substance, is satisfactory with regard to stability and has acceptable other physical parameters in addition.

### Disclosure of Invention

The invention has, as its object, a pharmaceutical composition containing the active substance atorvastatin in the form of an oblong-shaped tablet with the length of 5 to 22 mm and the width of 2 to 11 mm or a round tablet with the diameter of 3 to 16 mm, the core of which is constituted by compressed granulate and contains:
i. Atorvastatin and/or at least one physiologically acceptable salt thereof in the quantity of 5 to 10% by weight, related to pure atorvastatin;
ii. A base, which is meglumine in the quantity of 0.01 to 7% by weight;
iii. A pharmaceutically acceptable filler in the quantity of 20 to 90% by weight;
iv. A disintegrant in the quantity of 0.5 to 50% by weight;
provided with a coat which makes up 1 to 15% of the weight of the core, the selected base being uniformly distributed in the tablet core by means of spraying the same on the solid mixture in the granule production process.

Another object is represented by a process of manufacturing such composition, consisting in carrying out the following steps:
i. Mixing a mixture of atorvastatin, a filler and a disintegrant;
ii. Dissolving the base in a mixture of water and a C₁ to C₃ alcohol in the quantity of 10 : 90 to 90 : 10 (water/alcohol, by weight);
iii. Spraying the dry mixture with the base solution either in a masticating or fluidizing device;
iv. Adapting the size of particles of the resulting granulate, best by re-sieving to the granule size of 0.1 to 1.5 mm;
v. Adding other extragranular components to the granulate that will further improve its capability to be compressed into tablets;
vi. Compressing the mixture; and
vii. Applying a coat on the compressed tablets.

The invention also provides a composition produced by means of this procedure, which represents an advantageous solution of the subject matter of the invention.

### Detailed Description

Experiments with many various bases, used as stabilizing agents, have shown that a preferable base should get very quickly dissolved in the gastric fluid, which causes homogenization and eliminates its local concentrating and subsequent irritation of the mucous membrane. This has been achieved by uniform distribution of the base in the core of the tablet of the invention. This can be obtained by spraying the filler with a solution of the given base.

Out of the large number of bases that have been tested in this manner, meglumine or sodium hydroxide have turned out to be suitable. Although in the case of the hydroxide it would be the case of a strong base, just a low concentration could be used and with uniform distribution and ensuring of a quick decomposition of the tablet the possibility of a contact of NaOH with the stomach wall was eliminated.

For the realization of the invention the oblong shape of the tablet with the length of 5 to 22 mm and the width of 2 to 11 mm has proved to be advantageous. Benefits of the oblong shape are significant especially for higher doses of atorvastatin. However, the invention can also be realized in round tablets with the diameter of 3 to 16 mm.

It has also proved to be advantageous in the case of atorvastatin if the filler was mixed also with the active substance, the binder and at least a part of the disintegrant used and this mixture was sprayed with a solution of a base in a masticating or fluidizing device, thus generating a granulate that could be used for further production of tablets. The size of granules advantageously varies between 0.1 and 1.5 mm. However, for successful production of tablets the granulate must also contain dust fractions with the particle size lower than 0.1 mm. Preferably, the proportion of granules with the above mentioned size is 50 to 80% of the total granulate weight and 20 to 50% by weight of the dust particles. Individual components of the table are either located inside the granules or in the space between the granules.

By compressing the granulate and coating the same a coated tablet is produced whose properties may also be characterized by the tablet hardness of 50 to 300 N, disintegration in 10 to 600 s and the releasing rate at pH 4.5 at 75 rpm under conditions defined by Ph. Eur., wherein more than 60% of the active substance are released in 30 minutes.

The quantity of the base used varied between 0.01 and 7% by weight. Preferably, the base quantity was selected in the range between 0.1 and 5%, the quantity of meglumine is in the range of 0.5 to 5%. For example, 1% of meglumine proved to be suitable.

As the previous publications concluded, the release rate of the active substance - atorvastatin depends on its form present in the tablet. Experiments with various crystalline forms of atorvastatin showed in our case as well that it was not possible to achieve such a release rate as with amorphous atorvastatin. Hence the composition in accordance with the invention advantageously contains amorphous atorvastatin, especially preferably its calcium salt.

Stabilization of amorphous atorvastatin, as expected, has turned out to be more problematic than in the case of the crystalline form. Although both the bases ensure good stabilization, the product proved to be more stable if packed in an atmosphere with a lower partial pressure of oxygen. The partial pressure should be lower than at least 20 kPa. Significantly better results are achieved by further reductions of the partial pressure below 5, beneficially 2, preferably 1 kPa. If the pharmaceutical composition is packed in an atmosphere with the partial pressure of oxygen of 0.4 kPa or lower, no oxidation products appear at all.

The above mentioned need of quick decomposition of the tablet is related to the quantity and quality of the disintegrant used. For the realization of the invention it is necessary to use the disintegrant in a quantity of 0.5 to 50%, advantageously 3 to 25 % (weight percentage).

Further, using a combination of two types of disintegrants proved to be advantageous. The first one can be selected from the group of classical disintegrants, e.g. starch, pre-gelatinized starch, alginate, microcrystalline cellulose or low-substituted hydroxypropylcellulose. The other group contains so-called super disintegrants selected from the group of crosscarmellose, crosspovidone or the sodium salt of carboxymethyl starch.

Super disintegrants for the composition are selected in the quantity of 0.5 to 2.5% and the classical disintegrants in the quantity of 5 to 25% (weight percentage).

In a preferable embodiment crosscarmellose or crosspovidone is selected as the super disintegrant and low-substituted hydroxypropylcellulose as the classical disintegrant, which at the same time serves as the binder for the production of granulate. In a preferable embodiment the LH21 type of hydroxypropylcellulose was used.

The selection of the location of the disintegrant is also important for the effect of the subject matter of the invention. Envisaged is locating inside the granule or in the tablet in the space between individual granules. While in the former case it ensures disintegration of the granules themselves and releasing of the active substance, in the latter case it is disintegration of the tablet into individual granules. It is clear that both the processes participate in successful releasing of the effective substance.

It has been shown that for the case of the subject matter of the invention at least a part of the disintegrant used must be inside the granules. However, it was advantageous if all the disintegrant, i.e. both the types of the disintegrants used mentioned above were located inside the granules.

In addition to the total percentage of the base contained in the tablet, the weight proportion of this base to atorvastatin has turned out to be important, which varies between 1 : 100 and 1:2. In this case the selected proportion obviously depends on the selection of the particular base. The proportion of meglumine to atorvastatin is selected in the range of 1: 10 to 1: 2, while in the case of sodium hydroxide the proportion is 1: 100 to 10 : 100.

The principle of the invention is better elucidated in the following examples:

### Example 1 (comparative)

Coated tablets containing 10 mg of atorvastatin in the form of amorphous calcium salt with the composition specified in Table 1.

**Table 1**

| | Sample 010205 |
|---|---|
| | Quantity (mg) |
| Calcium salt of atorvastatin | 10.0 |
| Lactose monohydrate | 40.3 |
| Microcrystalline cellulose | 70.0 |
| Sodium salt of crosscarmellose | 4.5 |
| Hydroxypropylcellulose | 14.0 |
| Silicon dioxide | 0.5 |
| Magnesium stearate | 0.7 |

From the calcium salt of atorvastatin, lactose, microcrystalline cellulose and hydroxypropylcellulose a granulate was prepared by wet granulation, to which the sodium salt of crosscarmelose, silicon dioxide and magnesium stearate were added in an extra-granular manner after drying and sieving. This matter was compressed into tablets having the weight of 140 mg. The tablets were subsequently coated with standard varnish based on hydroxypropylmethylcellulose. The coated tablets were adjusted in Al/Al blister packing in a nitrogen atmosphere. The tablets were stored at the temperature of 40°C and the relative humidity of 75% for 6 months.

**Results of stability tests**

| Test - impurities, HPLC (%) | 0 months | 3 months | 6 months |
|---|---|---|---|
| Lactone | 0.11 | - | 1.07 |
| RTT 1.32 | 0.12 | - | 0.20 |
| Sum | 0.77 | - | 1.63 |

### Example 2

Optimization of the formulation - stabilization of the dosage form using meglumine as the basic substance.

Coated tables with the content of 10 mg of atorvastatin in the form of amorphous calcium salt with the composition specified in Table 2.

**Table 2**

| | Sample 020106 | Sample 030106 | Sample 060406 |
|---|---|---|---|
| | Quantity (mg) | Quantity (mg) | Quantity (mg) |
| Calcium salt of atorvastatin | 10.0 | 10.0 | 10.0 |
| Lactose monohydrate | 38.9 | 36.1 | 37.5 |
| Microcrystalline cellulose | 70.0 | 70.0 | 70.0 |
| Sodium salt of crosscarmellose | 4.5 | 4.5 | 4.5 |
| Hydroxypropylcellulose | 14.0 | 14.0 | 14.0 |
| Meglumine | 1.4 | 4.2 | 2.8 |
| Silicon dioxide | 0.5 | 0.5 | 0.5 |
| Magnesium stearate | 0.7 | 0.7 | 0.7 |

The calcium salt of atorvastatin was mixed with lactose, microcrystalline cellulose, hydroxypropylcellulose and a part of the sodium salt of crosscarmellose in a masticating device and was granulated with an alcoholic-aqueous solution of meglumine. The produced granulate was mixed with the sodium salt of crosscarmellose, silicon dioxide and magnesium stearate after drying and sieving. This matter was compressed into tablets with the weight of 140 mg. The tablets were subsequently coated with standard varnish based on hydroxypropylmethylcellulose. The coated tablets were adjusted in Al/Al blister packing in a nitrogen atmosphere. The tablets were stored at the temperature of 40°C and the relative humidity of 75% for 6 months.

**Results of stability tests - sample 020106**

| Test - impurities, HPLC (%) | 0 months | 3 months | 6 months |
|---|---|---|---|
| Lactone | 0.07 | 0.18 | 0.25 |
| RTT 1.32 | 0.06 | 0.06 | 0.06 |
| Sum | 0.55 | 0.61 | 0.67 |

**Results of stability tests - sample 030106**

| Test - impurities, HPLC (%) | 0 months | 3 months | 6 months |
|---|---|---|---|
| Lactone | 0.05 | 0.07 | 0.12 |
| RTT 1.32 | 0.06 | 0.05 | 0.06 |
| Sum | 0.51 | 0.51 | 0.58 |

**Results of stability tests - sample 060406**

| Test - impurities, HPLC (%) | 0 months | 3 months | 6 months |
|---|---|---|---|
| Lactone | 0.05 | 0.05 | 0.15 |
| RTT 1.32 | 0.07 | 0.08 | 0.07 |
| Sum | 0.50 | 0.56 | 0.64 |

### Example 3 (not within the scope of the claims)

Optimization of the formulation - stabilization of the dosage form using sodium hydroxide as the basic substance.

Coated tables with the content of 10 mg of atorvastatin in the form of amorphous calcium salt with the composition specified in Table 3.

**Table 3**

| | Sample 040106 |
|---|---|
| | Quantity (mg) |
| Calcium salt of atorvastatin | 10.0 |
| Lactose monohydrate | 39.9 |
| Microcrystalline cellulose | 70.0 |
| Sodium salt of crosscarmellose | 4.5 |
| Hydroxypropylcellulose | 14.0 |
| Sodium hydroxide | 0.4 |
| Silicon dioxide | 0.5 |
| Magnesium stearate | 0.7 |

The calcium salt of atorvastatin was mixed with lactose, microcrystalline cellulose, hydroxypropylcellulose and a part of the sodium salt of crosscarmellose in a masticating device and was granulated with an alcoholic-aqueous solution of sodium hydroxide. The produced granulate was mixed with the sodium salt of crosscarmellose, silicon dioxide and magnesium stearate after drying and sieving. This matter was compressed into tablets with the weight of 140 mg. The tablets were subsequently coated with standard varnish based on hydroxypropylmethylcellulose. The coated tablets were adjusted in Al/Al blister packing in a nitrogen atmosphere. The tablets were stored at the temperature of 40°C and the relative humidity of 75% for 6 months.

**Results of stability tests - sample 040106**

| Test - impurities, HPLC (%) | 0 months | 3 months | 6 months |
|---|---|---|---|
| Lactone | 0.05 | 0.18 | 0.33 |
| RTT 1.32 | 0.06 | 0.05 | 0.09 |
| Sum | 0.46 | 0.63 | 0.91 |

### Example 4

Optimization of the formulation - acceleration of release of the active substance from the dosage form at pH 4.5.

Coated tables with the content of 40 mg of atorvastatin in the form of amorphous calcium salt with the composition specified in Table 4

**Table 4**

| | Sample 071106 | Sample V/011106 | Sample V/090107 |
|---|---|---|---|
| | Quantity (mg) | Quantity (mg) | Quantity (mg) |
| Calcium salt of atorvastatin | 40.0 | 40.0 | 40.0 |
| Lactose monohydrate | 155.6 | 155.6 | 127.6 |
| Microcrystalline cellulose | 280.0 | 280.0 | 280.0 |
| Sodium salt of crosscarmellose | 6.0 + 12.0 | 28.0 + 0 | 56.0 + 0 |
| hydroxypropylcellulose | 56.0 | 56.0 | 56.0 |
| Meglumine | 5.6 | 5.6 | 5.6 |
| Silicon dioxide | 2.0 | 2.0 | 2.0 |
| Magnesium stearate | 2.8 | 2.8 | 2.8 |

The calcium salt of atorvastatin was mixed with lactose, microcrystalline cellulose, hydroxypropylcellulose and a part of the sodium salt of crosscarmellose in a masticating device and was granulated with an alcoholic-aqueous solution of sodium hydroxide. The produced granulate was mixed with the sodium salt of crosscarmellose, silicon dioxide and magnesium stearate after drying and sieving. This matter was compressed into tablets with the weight of 560 mg.

The tablets produced this way were subject to a dissolution test at pH 4.5 and 75 rpm.

The results of releasing of the active substance from the tablets (%) are specified in the following Table:

| | 10 min | 20 min | 30 min | 45 min |
|---|---|---|---|---|
| Sample 071106 | 54.3 | 59.3 | 62.7 | 65.5 |
| Sample V/011106 | 74.1 | 80.6 | 82.7 | 84.8 |
| Sample V/090107 | 80.5 | 85.5 | 87.0 | 87.9 |

## Claims

1. A pharmaceutical composition containing the active substance atorvastatin, designed for treatment and prevention of cardiovascular diseases, ***characterized in* that** it the form of oblong-shaped tablets with the length of 5 to 22 mm and the width of 2 to 11 mm or round tablets with the diameter of 3 to 16 mm, the core of which is constituted of compressed granulate and contains:
i. Atorvastatin and/or at least one physiologically acceptable salt thereof in the quantity of 5 to 10% by weight, related to pure atorvastatin;
ii. A base, which is meglumine, in the quantity of 0.01 to 7% by weight;
iii. A pharmaceutically acceptable filler in the quantity of 20 to 90% by weight; and
iv. A disintegrant in the quantity of 0.5 to 50% by weight;
provided with a coat that makes up 1 to 15% of the weight of the core, said base being uniformly distributed in the tablet core by means of spraying the same on the solid mixture in the granule production process.

2. The composition in accordance with claim 1, ***characterized in* that** it is a tablet having an oblong shape.

3. The pharmaceutical composition in accordance with claims 1 or 2, ***characterized in* that** it contains atorvastatin and/or its salt in an amorphous form.

4. The pharmaceutical composition in accordance with any of the preceding claims, ***characterized in* that** 50 to 80% by weight of the granulate consist of granules and their size varies in the range of 0.1 to 1.5 mm and 20 to 50% of the granulate are dust fractions smaller than 0.1 mm.

5. The pharmaceutical composition in accordance with any of the preceding claims,
***characterized in* that** its hardness value is 50 to 300 N, disintegration value is 10 to 600 s and more than 60% of the active substance are released in 30 minutes at pH 4.5 at 75 rpm under conditions defined by Ph. Eur.

6. The pharmaceutical composition in accordance with any of the preceding claims, ***characterized in* that** is it packed and kept in blister packing or a vial.

7. The pharmaceutical composition in accordance with claim 6, ***characterized in* that** a partial pressure of oxygen Pₒ lower than 20 kPa is maintained in the respective package.

8. The pharmaceutical composition in accordance with claim 7, ***characterized in* that** a partial pressure of oxygen Pₒ lower than 5 kPa is maintained in the respective package.

9. The pharmaceutical composition in accordance with claim 8, ***characterized in* that** a partial pressure of oxygen Pₒ lower than 2 kPa is maintained in the respective package.

10. The pharmaceutical composition in accordance with claim 9, ***characterized in* that** a partial pressure of oxygen Pₒ lower than 1 kPa is maintained in the respective package.

11. The pharmaceutical composition in accordance with claim 10, ***characterized in* that** a partial pressure of oxygen Pₒ lower than 0.4 kPa is maintained in the respective package.

12. The pharmaceutical composition in accordance with any of the preceding claims, ***characterized in* that** a part of the disintegrant used is located inside the granules compressed into the tablet and the second part is located in the space between granules in the tablet.

13. The pharmaceutical composition in accordance with claims 1 to 11, ***characterized in* that** all the disintegrant is located inside the granules compressed into tablets.

14. The pharmaceutical composition in accordance with any of the preceding claims, ***characterized in* that** a combination of two types of disintegrants is used, i.e. classical disintegrants from the group of starches, pre-gelatinized starches, alginates, microcrystalline cellulose or low-substituted hydroxypropylcellulose and a so-called super disintegrants from the group of crosscarmellose, crosspovidone or the sodium salt of carboxymethyl starch.

15. The pharmaceutical composition in accordance with claim 14, ***characterized in* that** it is a combination of the disintegrants crosscarmellose or crosspovidone and low-substituted hydroxypropylcellulose .

16. The pharmaceutical composition in accordance with claim 15, ***characterized in* that** crosscarmellose or crosspovidone is present in the quantity of 0.5 to 25% by weight and low-substituted hydroxypropylcellulose is present in the quantity of 5 to 25% by weight.

17. The pharmaceutical composition in accordance with claim 16, ***characterized in* that** crosscarmellose or crosspovidone is present in the quantity of 1.5 to 10% by weight and low-substituted hydroxypropylcellulose is present in the quantity of 7 to 15% by weight.

18. The pharmaceutical composition in accordance with any of the preceding claims, ***characterized in* that** meglumine is in a proportion to atorvastatin of 1: 2 to 1: 10.

19. The pharmaceutical composition in accordance with claim 18, ***characterized in* that** it has been obtained by a procedure comprising:
i. Mixing a mixture of atorvastatin, a filler, a disintegrant and a binder;
ii. Dissolving meglumine in a mixture of water and a C₁ to C₃ alcohol in a weight ratio of 10 : 90 to 90 : 10;
iii. Spraying the dry mixture with the solution of meglumine in a masticating or fluidizing device;
iv. Adapting the size of particles of the resulting granulate, preferably by re-sieving to the granule size of 0.1 to 1.5 mm;
v. Adding other extragranular components to the granulate;
vi. Compressing the mixture;
vii. Applying a coat on the compressed tablets.

20. The pharmaceutical composition in accordance with any of the preceding claims, ***characterized in* that** the film-forming substance hydroxypropylmethylcellulose, hydroxypropylcellulose, acrylate-based substances or their mixtures are used in the coat.

## Patentansprüche

1. Pharmazeutische Zusammensetzung, die den Wirkstoff Atorvastatin enthält, hergerichtet für die Behandlung und Prävention von Herz-Kreislauf-Erkrankungen, **dadurch gekennzeichnet, dass** sie die Form von länglich geformten Tabletten mit der Länge von 5 bis 22 mm und der Breite von 2 bis 11 mm oder runden Tabletten mit dem Durchmesser von 3 bis 16 mm hat, deren Kern aus komprimiertem Granulat gebildet ist und enthält:
i. Atorvastatin und/oder mindestens ein physiologisch annehmbares Salz davon in der Menge von 5 bis 10 Gew.%, bezogen auf reines Atorvastatin;
ii. eine Base, die Meglumin ist, in der Menge von 0,01 bis 7 Gew.%;
iii. einen pharmazeutisch annehmbaren Füllstoff in der Menge von 20 bis 90 Gew.%; sowie
iv. ein Zerfallsmittel in der Menge von 0,5 bis 50 Gew.%;
versehen mit einer Hülle, die 1 bis 15 % des Gewichts der Kerns ausmacht, wobei die Base gleichmäßig im Tablettenkern verteilt ist, **dadurch**, dass diese im Granalienherstellungsverfahren auf die feste Mischung aufgesprüht wird.

2. Zusammensetzung gemäß Anspruch 1, **dadurch gekennzeichnet, dass** sie eine Tablette mit einer länglichen Form ist.

3. Pharmazeutische Zusammensetzung gemäß Anspruch 1 oder 2, **dadurch gekennzeichnet, dass** sie Atorvastatin und/oder sein Salz in amorpher Form enthält.

4. Pharmazeutische Zusammensetzung gemäß einem der vorhergehenden Ansprüche, **dadurch gekennzeichnet, dass** 50 bis 80 Gew.% des Granulats aus Granalien besteht, und deren Größe im Bereich von 0,1 bis 1,5 mm variiert und 20 bis 50 % des Granulats Staubfraktionen kleiner als 0,1 mm sind.

5. Pharmazeutische Zusammensetzung gemäß einem der vorhergehenden Ansprüche, **dadurch gekennzeichnet, dass** ihr Härtewert 50 bis 300 N, ihr Zerfallwert 10 bis 600 s ist, und mehr als 60 % des Wirkstoffs in 30 Minuten bei pH 4,5 bei 75 U/min unter durch Ph. Eur. definierten Bedingungen freigesetzt werden.

6. Pharmazeutische Zusammensetzung gemäß einem der vorhergehenden Ansprüche, **dadurch gekennzeichnet, dass** sie verpackt ist und in Blister-Verpackung oder einem Fläschchen aufbewahrt wird.

7. Pharmazeutische Zusammensetzung gemäß Anspruch 6, **dadurch gekennzeichnet, dass** ein Partialdruck von Sauerstoff Pₒ von weniger als 20 kPa in der entsprechenden Verpackung aufrechterhalten wird.

8. Pharmazeutische Zusammensetzung gemäß Anspruch 7, **dadurch gekennzeichnet, dass** ein Partialdruck von Sauerstoff Pₒ von weniger als 5 kPa in der entsprechenden Verpackung aufrechterhalten wird.

9. Pharmazeutische Zusammensetzung gemäß Anspruch 8, **dadurch gekennzeichnet, dass** ein Partialdruck von Sauerstoff Pₒ von weniger als 2 kPa in der entsprechenden Verpackung aufrechterhalten wird.

10. Pharmazeutische Zusammensetzung gemäß Anspruch 9, **dadurch gekennzeichnet, dass** ein Partialdruck von Sauerstoff Pₒ von weniger als 1 kPa in der entsprechenden Verpackung aufrechterhalten wird.

11. Pharmazeutische Zusammensetzung gemäß Anspruch 10, **dadurch gekennzeichnet, dass** ein Partialdruck von Sauerstoff Pₒ von weniger als 0,4 kPa in der entsprechenden Verpackung aufrechterhalten wird.

12. Pharmazeutische Zusammensetzung gemäß einem der vorhergehenden Ansprüche, **dadurch gekennzeichnet, dass** sich ein Teil des verwendeten Zerfallsmittels innerhalb der Granalien befindet, die in die Tablette komprimiert sind, und dass sich der zweite Teil in dem Raum zwischen den Granalien in der Tablette befindet.

13. Pharmazeutische Zusammensetzung gemäß Ansprüchen 1 bis 11, **dadurch gekennzeichnet, dass** sich das gesamte Zerfallsmittel innerhalb der Granalien befindet, die in die Tabletten komprimiert sind.

14. Pharmazeutische Zusammensetzung gemäß einem der vorhergehenden Ansprüche, **dadurch gekennzeichnet, dass** eine Kombination von zwei Arten von Zerfallsmitteln verwendet wird, d.h. klassische Zerfallsmittel aus der Gruppe der Stärken, vorgelatinierten Stärken, Alginate, mikrokristallinen Cellulose oder niedrig-substituierten Hydroxypropylcellulose, sowie sogenannte Superzerfallsmittel aus der Gruppe von Croscarmellose, Crospovidon oder dem Natriumsalz von Carboxymethylstärke.

15. Pharmazeutische Zusammensetzung gemäß Anspruch 14, **dadurch gekennzeichnet, dass** sie eine Kombination der Zerfallsmittel Croscarmellose oder Crospovidon und niedrig-substituierter Hydroxypropylcellulose ist.

16. Pharmazeutische Zusammensetzung gemäß Anspruch 15, **dadurch gekennzeichnet, dass** Croscarmellose oder Crospovidon in der Menge von 0,5 bis 25 Gew.% vorhanden ist und niedrig-substituierte Hydroxypropylcellulose in der Menge von 5 bis 25 Gew.% vorhanden ist.

17. Pharmazeutische Zusammensetzung gemäß Anspruch 16, **dadurch gekennzeichnet, dass** Croscarmellose oder Crospovidon in der Menge von 1,5 bis 10 Gew.% vorhanden ist und niedrig-substituierte Hydroxypropylcellulose in der Menge von 7 bis 15 Gew.% vorhanden ist.

18. Pharmazeutische Zusammensetzung gemäß einem der vorhergehenden Ansprüche, **dadurch gekennzeichnet, dass** Meglumin in einem Verhältnis zu Atorvastatin von 1:2 bis 1:10 vorhanden ist.

19. Pharmazeutische Zusammensetzung gemäß Anspruch 18, **dadurch gekennzeichnet, dass** sie durch ein Verfahren gewonnen wurde, das umfasst:
i. Mischen einer Mischung von Atorvastatin, einem Füllstoff, einem Zerfallsmittel und einem Bindemittel;
ii. Auflösen von Meglumin in einer Mischung von Wasser und einem C₁-C₃-Alkohol in einem Gewichtsverhältnis von 10:90 bis 90:10;
iii. Besprühen der trockenen Mischung mit der Lösung von Meglumin in einem Mastizier- oder Verflüssigungsgerät;
iv. Anpassen der Größe der Partikel des sich ergebenden Granulats, vorzugsweise durch Sieben auf die Granaliengröße von 0,1 bis 1,5 mm;
v. Zugeben von anderen extragranulären Bestandteilen zu dem Granulat;
vi. Komprimieren der Mischung;
vii. Auftragen einer Beschichtung auf die komprimierten Tabletten.

20. Pharmazeutische Zusammensetzung gemäß einem der vorhergehenden Ansprüche, **dadurch gekennzeichnet, dass** der filmbildende Stoff Hydroxypropylmethylcellulose, Hydroxypropylcellulose, Acrylat-basierte Stoffe oder deren Mischungen in der Beschichtung verwendet werden.

## Revendications

1. Une composition pharmaceutique contenant la substance active atorvastatine destinée au traitement et à la prévention de maladies cardio-vasculaires, **caractérisée en ce qu'**elle est sous la forme de comprimés de forme oblongue dont la longueur est de 5 à 22 mm et la largeur de 2 à 11 mm, ou de comprimés ronds dont le diamètre est de 3 à 16 mm, le noyau desquels étant constitué de granules compressés et contient :
i. de l'atorvastatine et/ou au moins un sel physiologiquement acceptable en dérivant en une quantité de 5 à 10 % en poids par rapport à l'atorvastatine pure ;
ii. une base, laquelle est la méglumine, en une quantité de 0,01 à 7 % en poids ;
iii. une charge pharmaceutiquement acceptable en une quantité de 20 à 90 % en poids ; et
iv. un délitant en une quantité de 0,5 à 50 % en poids ;
fournis avec un revêtement qui forme de 1 à 15 % du poids du noyau, ladite base étant distribuée de façon uniforme dans le noyau du comprimé par pulvérisation de celle-ci dans un mélange solide lors du procédé de production de granules.

2. La composition selon la revendication 1, **caractérisée en ce qu'**elle est un comprimé ayant une forme oblongue.

3. La composition pharmaceutique selon les revendications 1 ou 2, **caractérisée en ce qu'**elle contient de l'atorvastatine et/ou son sel sous une forme amorphe.

4. La composition pharmaceutique selon l'une quelconque des revendications précédentes, **caractérisée en ce que** 50 à 80 % en poids du granulat consiste en des granules et que leur dimension varie dans la gamme de 0,1 et 1,5 mm et **en ce que** 20 à 50 % des granules sont des fractions de poussières inférieures à 0,1 mm.

5. La composition pharmaceutique selon l'une quelconque des revendications précédentes, **caractérisée en ce que** sa valeur de dureté est de 50 à 300 N, **en ce que** la valeur de désintégration est de 10 à 600 s et **en ce que** plus de 60 % de la substance active sont libérés en 30 minutes à un pH de 4,5 à 75 tours par minute, sous des conditions définies par Ph. Eur.

6. La composition pharmaceutique selon l'une quelconque des revendications précédentes, **caractérisée en ce qu'**elle est contenue dans un emballage et maintenue dans un emballage de type blister ou un flacon.

7. La composition pharmaceutique selon la revendication 6, **caractérisée en ce qu'**une pression partielle en oxygène P₀ inférieure à 20 kPa est maintenue dans l'emballage respectif.

8. La composition pharmaceutique selon la revendication 7, **caractérisée en ce qu'**une pression partielle en oxygène P₀ inférieure à 5 kPa est maintenue dans l'emballage respectif.

9. La composition pharmaceutique selon la revendication 8, **caractérisée en ce qu'**une pression partielle en oxygène P₀ inférieure à 2 kPa est maintenue dans l'emballage respectif.

10. La composition pharmaceutique selon la revendication 9, **caractérisée en ce qu'**une pression partielle en oxygène P₀ inférieure à 1 kPa est maintenue dans l'emballage respectif.

11. La composition pharmaceutique selon la revendication 10, **caractérisée en ce qu'**une pression partielle en oxygène P₀ inférieure à 0,4 kPa est maintenue dans l'emballage respectif.

12. La composition pharmaceutique selon l'une quelconque des revendications précédentes, **caractérisée en ce qu'**une partie du délitant utilisé est située à l'intérieur des granules compressées dans le comprimé et la deuxième partie est située dans l'espace entre les granules du comprimé.

13. La composition pharmaceutique selon les revendications 1 à 11, **caractérisée en ce que** la totalité du délitant est située à l'intérieur des granules compressées des comprimés.

14. La composition pharmaceutique selon l'une quelconque des revendications précédentes, **caractérisée en ce qu'**une combinaison de deux types de délitants est utilisée, c'est-à-dire des délitants classiques du groupe des amidons, des amidons pré-gélatinisés, des alginates, de la cellulose microcristalline ou de l'hydroxypropylcellulose faiblement substituée et un agent appelé super délitant du groupe des crosscarmellose, crosspovidone ou sel de sodium de carboxyméthylamidon.

15. La composition pharmaceutique selon la revendication 14, **caractérisée en ce qu'**elle est une combinaison de délitants crosscarmellose ou crosspovidone et d'hydroxypropylcellulose faiblement substituée.

16. La composition pharmaceutique selon la revendication 15, **caractérisée en ce que** la crosscarmellose ou la crosspovidone sont présentes en une quantité de 0,5 à 25 % en poids et l'hydroxypropylcellulose faiblement substituée est présente en une quantité de 5 à 25 % en poids.

17. La composition pharmaceutique selon la revendication 16, **caractérisée en ce que** la crosscarmellose ou la crosspovidone sont présentes en une quantité de 1,5 à 10 % en poids et l'hydroxypropylcellulose faiblement substituée est présente en une quantité de 7 à 15 % en poids.

18. La composition pharmaceutique selon l'une quelconque des revendications précédentes, **caractérisée en ce que** la méglumine est employée en une proportion par rapport à l'atorvastatine de l'ordre de 1:2 à 1:10.

19. La composition pharmaceutique selon la revendication 18, **caractérisée en ce qu'**elle a été obtenue par un procédé impliquant :
i. le mélange d'un mélange formé d'atorvastatine, d'une charge, d'un délitant et d'un liant ;
ii. la dissolution de la méglumine dans un mélange d'eau et d'un alcool en C₁ à C₃ dans un rapport en poids de 10 :90 à 90 :10 ;
iii. la pulvérisation du mélange sec avec la solution de méglumine dans un dispositif de mastication et de fluidisation ;
iv. l'adaptation de la dimension des particules du granulat résultant, de préférence par re-tamisage à une dimension granulaire de 0,1 à 1,5 mm ;
v. l'addition d'autres composants extra-granulaires au granulat ;
vi. la compression du mélange ;
vii. l'application d'un revêtement sur les comprimés compressés.

20. La composition pharmaceutique selon l'une quelconque des revendications précédentes, **caractérisée en ce que** la substance filmogène d'hydroxypropylméthylcellulose, d'hydroxypropylcellulose, de substances à base d'acrylate ou leurs mélanges, sont utilisées dans le revêtement.
